# EUROPEAN PATENT APPLICATION

(11) **EP 2 886 662 A1**
(43) Date of publication of application: **24.06.2015**
(21) Application number: 13306780.1
(22) Date of filing: 19.12.2013
(51) Int. Cl.: C12Q 1/68

(54) **AP5B1 as a new marker for moderate to severe acne**

(71) Applicant: King's College London, London WC2R 2LS (GB); Galderma Research & Development, 06410 Biot (FR)
(72) Inventor: Barker, Jonathan, London, WC2R 2LS (GB); Smith, Catherine, London, WC2R 2LS (GB); Trembath, Richard, London, WC2R 2LS (GB); Carlavan, Isabelle, 06410 Biot (FR)
(74) Representative: Starck-Loudes, Anne-Caroline

(57) **Abstract**

The invention relates to the identification of Adaptor-Related Protein Complex 5, subunit 1 (AP5B1) as an acne marker and to screen of compounds which modulate (AP5B1) as well as their use for treating acne. It also relates to methods for the in vitro diagnosis or in vitro prognosis for acne vulgaris.

## Description

The invention relates to the identification of Adaptor-Related Protein Complex 5, subunit 1 (AP5B1) as an acne marker and to screen of compounds which modulate (AP5B1) as well as their use for treating acne. It also relates to methods for the in vitro diagnosis or in vitro prognosis for acne vulgaris.

Acne is a skin condition which results from the occlusion of the upper end and also of the internal part of the pilosebaceous canal owing to abnormal keratinocyte multiplication, and the androgenic hormone hyperactivity which often appears during puberty, which causes a considerable increase in seborrhea in the sebaceous glands. The obstruction of the pilosebaceous canal causes the formation of comedones or microcysts, accompanied by proliferation of Propionibacterium acnes bacteria in the obstructed pilosebaceous follicles.
This condition, which is particularly common in adolescents, is accompanied by an inflammatory reaction of the skin that may be in the form of papules or pustules generally located in the superficial dermis. In certain cases, the inflammatory reaction may reach the deep dermis, forming nodules and macrocysts.

Acne is the most common skin condition affecting millions of people worldwide. Patients with severe acne frequently face significant psychological and emotional problems due to the scarring associated with the disease. The pathogenesis of acne vulgaris is complex and incompletely understood.

### AP5B1

AP5B1, or Adaptor-Related Protein Complex 5, Beta 1 Subunit [NM_138368.4 (mRNA) NP_612377 (protein)], appears to be a large subunit of an adaptor protein complex, AP5, that has a role in endocytosis.
AP-5 is the most recently identified and the least well characterized of the heterotetrameric adaptor protein (AP) complexes. AP complexes facilitate the trafficking of cargo from one membrane compartment of the cell to another by recruiting other proteins to particular types of vesicles. Despite a sequence divergence with other AP members, AP-5 shares features with the other AP complexes such as four subunits, which are similar in size to the corresponding subunit in other APs.
Mutations in AP-5 can cause congenital neurological disorders in humans (Slabicki M, Theis M, Krastev DB, Samsonov S, Mundwiller E,Junqueira M, Paszkowski-Rogacz M, Teyra J, Heninger AK, Poser I, Prieur F, Truchetto J, Confavreux C, Marelli C, Durr A, et al.A genome-scale DNA repair RNAi screen identifies SPG48 as a novel gene associated with hereditary spastic paraplegia. PLoS Biol 2010).
The depletion of endogenous protein by using siRNA leads to a striking change in the localisation of the cation-independent mannose 6-phosphate receptor (CIMPR), a receptor for lysosomal hydrolases that cycles between the Trans-Golgi-Network and endosomes. AP-5 is localized on late endosomal/lysosomal membranes and the pathway it acts on is unclear. Hirst J, Barlow LD, Francisco GC, Sahlender DA, Seaman MN, Dacks JB, Robinson MS. The fifth adaptor protein complex. PLoS Biol. 2011 Oct; 9(10))
However, any defect in trafficking can lead to a number of endosome and lysosome related human diseases (e.g. Hermansky-Pudlak syndrome; Spatic paraplegia) (Dell'Angelica EC, Shotelersuk V, Aguilar RC, Gahl WA, Bonifacino JS. Altered trafficking of lysosomal proteins in Hermansky-Pudlak syndrome due to mutations in the beta 3A subunit of the AP-3 adaptor. Mol Cell. 1999 Jan; 3(1):11-21).
AP-5 functions as a cargo adaptor, missorting of one or more cargo proteins, not yet identified, leading to abnormal endosomal / lysosomal trafficking of molecules that can affect skin homeostasis.
In the context of acne, a defect in AP5 function can contribute to abnormal follicular differentiation with increased cornification, enhanced sebaceous gland activity with hyperseborrhea, abnormal healing. Indeed, endocytic pathway regulates signaling of receptors which are involved in skin diseases such as EGFR, BMP receptors, TGFB receptors (Ceresa BP. Spatial regulation of epidermal growth factor receptor signaling by endocytosis. Int J Mol Sci. 2012 Dec 20; 14(1):72-87; Balogh P, Katz S, Kiss AL. The role of endocytic pathways in TGF-β signaling. Pathol Oncol Res. 2013 Apr; 19(2):141-8; Hartung A, Bitton-Worms K, Rechtman MM, Wenzel V, Boergermann JH, Hassel S,Henis YI, Knaus P. Different routes of bone morphogenic protein (BMP) receptor endocytosis influence BMP signaling. Mol Cell Biol. 2006 Oct; 26(20):7791-805).

For the first time, the applicant proposes with experimental evidences, particularly Genome Wide Association Study results, AP5B1 as a marker for diagnosing moderate to severe acne vulgaris and to modulate its expression for treating acne vulgaris.

Thus, the invention is relating to the use of the DNA or the mRNA encoding AP5B1 or the corresponding protein, as markers for acne vulgaris.

The invention also provides a method for diagnosing or determining a predisposition to acne and/or assessing the severity of acne in a patient comprising the step of assessing: (a) the level of expression; (b) the activity; or (c) the sequence of AP5B1 gene, promoter and/or expression product.

For example, the method for diagnosing acne, may comprise the following steps:
a) analysing the level of expression or the activity or the sequence of AP5B1 including exons, introns, upstream and downstream non-coding regions involved in the regulation of AP5B1 expression, in a biological sample from an individual,
b) analysing the level of expression or the activity or the sequence exons, introns, upstream and downstream non-coding regions involved in the regulation of AP5B1 expression in the regulation of AP5B1 expression, in a biological sample from a healthy individual,
c) comparing the difference in level of expression or activity or in sequence of AP5B1 including exons, introns, upstream and downstream non-coding regions involved in the regulation of AP5B1 expression to healthy individual,
d) the difference of step c) is indicative of acne disease or a predisposition to acne, thus diagnosing acne vulgaris.

The invention provides a method for monitoring the progression of acne, comprising the following steps:
a) taking a biological sample from the individual,
b) analysing level of expression or the activity or the sequence of AP5B1 including exons, introns, upstream and downstream non-coding regions involved in the regulation of AP5B1 expression in a sample taken and in which a variation in the expression or activity or in the sequence of AP5B1 including introns, upstream and downstream non-coding regions involved in the regulation of AP5B1 expression is an indicator of the progression of acne.
Progression of acne may be from a predominantly comedonal to a more inflammatory dominated state, it may also mean progression towards specific acne subtypes, like nodulocystic acne or acne conglobata for example. Progression might also occur in the other direction, from a more severe to a less severe form of acne.

The invention provides also a method for monitoring the efficacy of a treatment intended for treating acne, comprising the following steps:
a) administering the desired treatment to the individual identified as having one or more of the symptoms of acne,
b) taking a biological sample from the individual,
c) analysing the level of expression or the activity or the sequence of AP5B1 including exons, introns, upstream and downstream non-coding regions involved in the regulation of AP5B1 expression, in which a variation in the expression, activity or sequence of this marker is an indicator in the treatment of acne.
Another embodiment of the present invention is in vitro screening method of modulators of AP5B1 for treating acne, comprising determining the capacity of said candidate to restore physiological expression levels and/or biological function of AP5B1.

Methods of diagnosing and monitoring typically involve comparing quantitative or semi-quantitative values (of expression or activity level), or sequence, obtained with the test sample, with control values (of expression or activity level)or control sequence.

More specifically, the invention provides an in vitro screening method of AP5B1 modulators for the identification of drug candidates, comprising the following steps:
a) Collecting at least one biological sample;
b) Contacting at least one sample or a mixture of samples with one or more drug candidates to be tested;
c) Detecting the expression or biological function of AP5B1 in the biological samples or mixture obtained in b);
d) Selecting drug candidates, which are capable of restoring physiological expression and/or biological function of AP5B1 measured in said samples or mixtures obtained in b) and comparing the levels with a sample not mixed with the drug candidate (s).

For step a), in an alternative embodiment, two biological samples are collected, one mimics acne lesion and one mimics the healthy condition.
The invention relates also to the use of modulators identified by screening methods as defined above for the preparation of a composition for treating acne and/or acne associated disorder such as comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne and also more largely, acne associated disorders (e.g. hyperseborrhoea).

For the purpose of the present invention, the term "marker" or "biological marker" denotes a biological marker associated with the presence or with the absence of a particular pathological state. The biological markers are in particular proteins, mRNAs or DNAs.

The term "level of expression" or "expression" means the level of mRNAs or proteins encoded by the gene marker.
The expression level analysis or detection can be performed by any suitable method, known to those skilled in the art, such as western blotting, IHC, mass spectrometry (Maldi-TOF and LC/MS analyses), radioimmunoassay (RIA), Elisa or any other method known to those skilled in the art or else by assaying the mRNA according to the methods customarily known to those skilled in the art. The techniques based on the hybridization of mRNA with specific nucleotide probes are the most customary (Northern blotting, RT-PCR (Reverse Transcriptase Polymerase Chain Reaction), quantitative RT-PCR (qRT-PCR), RNase protection).
The sequence analysis can be performed by any suitable method, known to those skilled in the art, such as next-generation sequencing (NGS).

For the screening, biological samples are transfected cells containing reporter gene operably under the control of a promoter (totally or partially) controlling the expression of AP5B1 gene. Therefore step c) above consists in measuring the AP5B1 expression of the reporter gene.
The reporter gene may encode an enzyme that with its corresponding substrate, provides coloured product(s) such as CAT (chloramphenicol acetyltransferase), GAL (beta galactosidase), or GUS (beta glucuronidase). It might be either luciferase or GFP (Green Fluorescent Protein) gene. Reporter gene protein dosage or its activity is typically assessed by colouring, fluorometric or chemoluminescence methods.

Biological samples are also cells expressing the gene of interest and the step c) above consists to measure the activity of the gene product.

Any kind of cell is suitable for the invention. Cells may endogenously express the said gene like keratinocytes. Organs may be suitable for the instant invention, from animal or human origin like skin.
Transformed cells by heterologous nucleic acid encoding the gene expression product of interest might be suitable. Preferably the said nucleic acid is from animal (preferred mammal) or human origin. A large variety of host cells is suitable for the invention and in particular Cos-7, CHO, BHK, 3T3, HEK293 cells. Cells may be transiently or permanently transfected by a nucleic acid of interest with a well-known by those skilled in the art method and for instance calcium phosphate precipitation, DEAE-dextran, liposome, virus, electroporation or microinjection.
The gene expression of step c) is determined with the same techniques quoted above.

The compounds to be tested are any kind of compounds, from natural or synthetic source. As synthetic compounds they might be chemically synthesized or from a chemical compound data bank, with a defined structure or non-characterized or present in a mixture of compounds. Molecules restoring the physiological expression of AP5B1 or its biological activity or biological function can also be provided by all systems and methods modulating the expression of AP5B1 such as methods of gene replacement, gene therapy and delivery of therapeutic protein, comprising AP5B1 (miRNA, mRNA, DNA, protein).

In the context of the invention, the biological sample corresponds to any type of sample taken from an individual, and can be a tissue sample or a fluid sample, such as blood, lymph or interstitial fluid.

According to one particular and preferred embodiment, the sample is a biopsy of varying size (preferably from 1 to 6 mm in diameter), or a skin sample taken by means of tape stripping, such as with D-Squames, according to the method described in Wong R et al., "Analysis of RNA recovery and gene expression in the epidermis using non-invasive tape stripping"; J Dermatol Sci.2006 Nov; 44(2):81-92; or in Benson NR, et al., "An analysis of select pathogenic messages in lesional and non-lesional psoriatic skin using non-invasive tape harvesting". J Invest Dermatol. 2006 Oct; 126(10): 2234-41; or else in Wong R et al., "Use of RT-PCR and DNA microarrays to characterize RNA recovered by non-invasive tape harvesting of normal and inflamed skin". J Invest Dermatol. 2004 Jul; 123(1):159-67. According to the principle of tape stripping, the product used comprises a flexible translucent polymer support and an adhesive. The product is applied repeatedly to the skin of the patient, preferably until loss of adhesion. The sample obtained relates only to the content of the outermost layers of the epidermis. A method for analysing a protein content obtained in particular according to this sampling method is described in Patent Application WO2009/068825 (Galderma R&D) in order to monitor markers specific for a pathological skin condition and to orient the diagnosis. Since this method is rapid, non-invasive and relatively inexpensive for detecting the presence of, the absence of or the variation in certain proteomic markers, it is particularly preferred. This method is in particular characterized by mass spectrometry detection, ELISA or any other method known to the expert skilled in the art of protein quantification. Quantification is performed in the skin sample obtained on the flexible and adhesive support in order to detect at least one protein of which the presence, the absence or the variation in amount or in concentration compared with a standard value is associated with the presence, with the progression or with the absence of a particular pathological skin condition.

Another embodiment of the present invention is an in vitro screening method of AP5B1 modulators, comprising determining the capacity of said candidate to restore the physiological expression or the biological activity or the biological function, including the transactivation properties, of the proposed marker of the invention.

According to a further embodiment of the invention, biological samples are cells expressing the gene of interest and the step c) above consists to measure the activity of the gene product.

In another embodiment, the invention is related to the use of identified modulators with the described screening methods for the preparation of a composition for treating acne and/or acne associated disorders. Preferably the identified modulator is a polynucleotide, a polypeptide, an antibody or a small organic molecule.

The invention is intended for treating acne. By acne it is understood, all acne forms especially simple acne, comedonic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne and medication-related acne and also more largely, acne associated disorders (e.g. hyperseborrhoea)

The examples, which follow, illustrate the invention without limiting the scope thereof.

### Legend to the Figure:

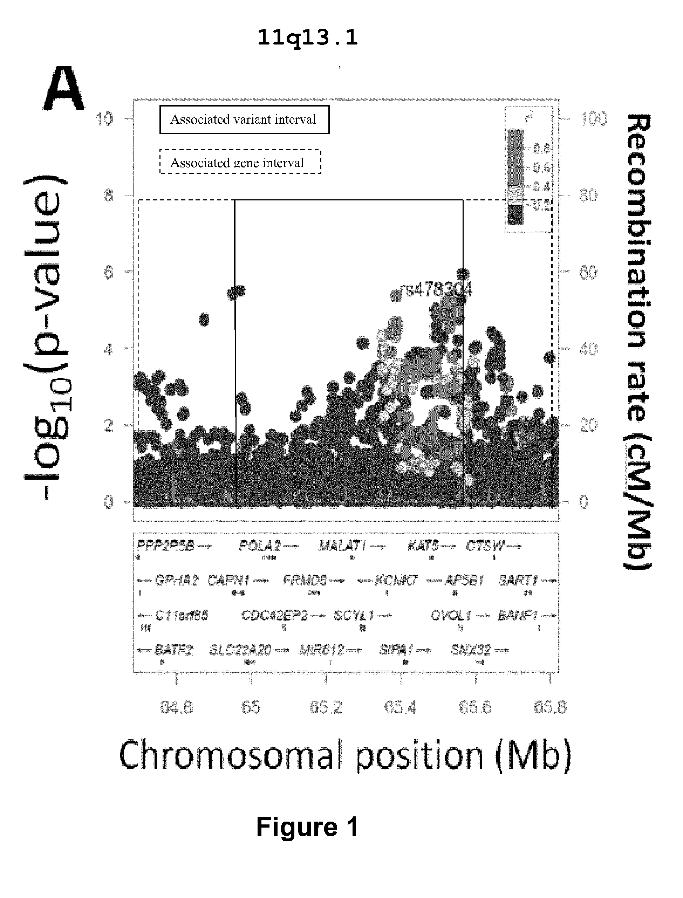

**Figure 1** represents the regional association plots of Chr. 11q13.

### Example 1: GWAS study.

Since, twin and family studies indicate that a family history doubles the risk of significant acne, we performed the first GWAS in moderate-to-severe acne in order to identify the predisposing genetic architecture.

### Patients:

Patients had a diagnosis of acne vulgaris made by a trained dermatologist , with at least moderate severity as defined by the presence of nodulocystic disease and/or Leeds Grade >5 severity and/or requiring treatment with isotretinoin and or presence of severe forms of acne.
Samples: A total of 4,208 samples were assembled for this investigation, of which 2,001 samples from unrelated individuals of European ancestry were processed for the GWAS discovery set and 2,207 of which were processed for the second stage set. A total of 1,894 DNA samples, recruited from 17 centers from the UK were included in the GWAS discovery set, passed pre- and post-genotyping control filters (see below). A total of 2063 DNA samples were included in the second stage dataset. Phenotypic data and blood samples were collected after research ethics approval was received from each participating institution and after subjects had given written informed consent.
Controls: A total of 7,271 control individuals passed the quality control filters (see below). 5,139 individuals from the WTCCC2 common control set were used in the discovery GWAS. This included 2478 healthy blood donors from the United Kingdom Blood Service (UKBS) collection and 2661 individuals from the 1958 Birth Cohort (58C) dataset. 2,132 individuals enriched for no history of acne from the Twins UK registry were used in the second stage dataset.

### Quality control:

- Samples. We used plink and principal component analysis with Eigensoft to detect and exclude outlying individuals on the basis of call rate, heterozygosity, relatedness, sex mismatches and ancestry. We also excluded one of each pair of related individuals.
- SNPs. SNPs were excluded if the Fisher information for the allele frequency was not close to unity or for extreme departures from Hardy-Weinberg equilibrium (see below). Cluster plots of SNPs showing putative associations were inspected manually. The quality control measures excluded 251 cases and 169 controls. 31% (255193) non-overlapping SNPs were excluded during merging of case and control datasets, an additional 1.5% (8588) SNPs were excluded during QC in plink due to missingness thresholds of < 95% and/or Hardy Weinberg Equilibrium test with p < 10-6.
Statistical methods. We performed principal component analysis on a subset of 83,484 post-quality-control SNPs (none from the MHC region), selected so as to minimize the contribution from regions of extensive strong linkage desequilibrium and to ensure that only genome-wide effects were detected. Principal component scores were computed for the combined dataset of post-exclusion case and control samples. After inspection, the first four principal components did not lead to differentiation of individuals by geographical origin, suggesting negligible differences in ancestry within the dataset. However, a part of the acne cases clustered outside of the bulk of the plot without showing geographical or ethnical differences to the majority of cases. To guard against possible artefacts, we repeated the primary association analysis excluding these cases. Reassuringly, this yielded comparable association signals. As we expected genotyping-platform dependent differences between cases and controls, we chose to use the first four principal components. However, no single principal component showed significant differences between cases and control.
We used a logistic regression model with case or control status as the response variable, the first four principal components as covariates and the genotype at a particular SNP as the explanatory variable. Genome-wide association tests were carried out at each SNP with uncertainty in genotype calls modelled using missing data likelihoods as implemented in SNPTEST. Unless otherwise stated, we assumed that the change in the odds of case status due to each copy of the allele was multiplicative.
It has become standard practice in GWAS to refer to the odds ratio associated with a particular allele or haplotype, which we estimate as eß, where ß is the maximum likelihood estimate of the coefficient describing the effect of each predictor on the response in the assumed model. We note however that, as is true of this study and many others, where the controls are taken at random from the population without reference to disease status, ß is actually the log of the relative risk and not the log of the odds ratio.
Imputation was performed in a two-stage approach using phasing with ShapeIt and imputation using IMPUTE2 on the 1000 Genomes reference panel. We used frequentist conditional analyses to look for primary and secondary association signals at known and putative SNPs.
Selection of SNPs for replication was performed using the threshold of p value < 10-4 in the discovery set, prioritizing genotyped over imputed SNPs, SNPs with a minor allele frequency of > 0.02, as well as crossreferencing with lists of candidate genes that had arisen in transcription studies of acne biopsies and sebocyte cultures. We aimed to have at least two SNPs with r2 > 0.8 per region in case one should fail to be genotyped in the second stage cohort.
We used standard 'fixed-effect' meta-analysis techniques to analyse the second stage data. We fitted a logistic regression model for case or control status with no covariates at each SNP with a single parameter for the genetic effect (a multiplicative effect on the risk scale and an additive effect on the log-odds scale). As is typical for GWAS second stage studies which type a small number of SNPs, testing for possible substructure within populations was not possible. Figure 1 represents the regional association plots of Chr. 11q13.
The -log10 P values for the SNPs at the new locus are shown on the left y axis of each plot. SNPs are coloured based on their r2 with the labelled hit SNP which has the smallest P value in the region. r2 is calculated from the 1000 Genomes (March 2012) genotypes. The bottom section of each plot shows the fine scale recombination rates estimated from individuals in the 1000 Genomes population, and genes are marked by horizontal blue lines

Table 1 provides locus showing Genome-wide significant association with acne. Genome-wide significant associations identify a genomic region, from 64.8 Mb to 65.8 Mb, on 11q13.1 (rs478304) with P combined = 3.23 x 10-11. Among known genes encompassed in this region, AP5B1, which is very close to the lead SNP, is of major interest.

### Example 2: Analysis of the expression of AP5B1 in the epidermis and sebaceous glands of human normal skin.

This example provides an analysis of epidermis and sebaceous gland transcriptomes provided information on the physiological level of expression in the two compartments.

### Tissue biopsies:

Samples were obtained from face lifting surgery of five healthy donors. Epidermis and sebaceous glands were separated from dermis after enzymatic digestion. Sebaceous glands and epidermis were collected by dissection under a binocular microscope.

mRNA extraction, labelling and hybridization to probe arrays: The mRNA was isolated from samples using the RNeasy extraction kit (Qiagen Inc., Valencia,CA) and quality was evaluated using a 2100 Bioanalyser of Agilent. The mRNA expression was evaluated by a Gene Chip IVT labelling kit after the generation of double-stranded cDNA (i.e in vitro transcription process) using T7-oligo primer and the one cycle cDNA synthesis kit of Affymetrix. RNA was ethanol precipitated to concentrate the sample and then quantified using a spectrophotometer. Approximately 200 ng of total RNA of good quality [RNA indication number (RIN) ≥ 7] from each sample was used to generate double-stranded cDNA using a T7-oligo (dt) primer (one cycle cDNA synthesis kit, Affymetrix). Biotinylated cRNA, produced through in vitro transcription (Gene Chip IVT labelling kit, Affymetrix) was fragmented and hybridised to an Affymetrix human U133A 2.0 plus microarray. The arrays were processed on a Gene Chip Fluidics Station 450 and scanned on an Affymetrix Gene Chip Scanner (Santa Clara, CA).

### Statistical Analysis of mRNA expression based on Affymetrix gene chips:

The expression data from Affymetrix Gene Chips are normalized with RMA (Robust Multi-array Analysis) method. The raw intensity values are background corrected, log2 transformed and then quantile normalized. Next a linear model is fit to the normalized data to obtain an expression measure for each probe set on each array.

Table 2 collects data of AP5B1 mRNA expression measured by Affymetrix in the epidermis and sebaceous glands of five facial lifting of healthy human donors.

As shown in table 2, AP5B1 transcripts were clearly expressed in sebaceous glands as well as in epidermis in five healthy donors.

Altogether, the GWAS results and the expression of AP5B1 in human skin support a role of AP5B1 in acne pathology.

**Table 1**

| | | | | | RAF | | Discovery sample | | Second stage sample | | Combined |
|---|---|---|---|---|---|---|---|---|---|---|---|
| | | | | Risk allele | | | | | | | |
| Chr | rsID | Position | Gene of Interest | | Cases | Controls | P_{scan} | OR (95% Cl) | P_{2nd stage} | OR (95% Cl) | P_{com} |
| 11q13,1 | rs478304 | 65'494'260 | AP5B1 | T | 0,6 | 0,55 | 9,58x10⁻⁶ | 1,20(1,11-1,29) | 2,65x10⁻⁷ | 1,26(1,16-1,38) | 3,23x10⁻¹¹ |

**Table 2**

| | | | **Normalized Expression by RMA in Epidermis** | | | | | **Normalized Expre**s**sion by RMA in Sebaceous Gland** | | | | | | |
|---|---|---|---|---|---|---|---|---|---|---|---|---|---|---|
| **ProbeSet** | **GENE_ SYMBOL** | **TITLE** | **Donor 1** | **Donor 2** | **Donor 3** | **Donor 4** | **Donor 5** | **Donor 1** | **Donor 2** | **Donor 3** | **Donor 4** | **Donor 5** | **Mean Expression Epidermis** | **Mean Expression Sebaceaous Gland** |
| 231858_x_at | AP5B1 | adaptor-related protein complex 5, beta 1 subunit | 130 | 150 | 124 | 143 | 108 | 129 | 148 | 113 | 120 | 119 | 130 | 125 |

## Claims

1. Use of the DNA or the mRNA encoding AP5B1, or the corresponding protein, as markers for acne.

2. Method for the diagnosis or determining a predisposition to acne comprising the following steps:
a) analysing the level of expression or the activity or the sequence of AP5B1 including exons, introns, upstream and downstream non-coding regions involved in the regulation of AP5B1 expression in a biological sample from an individual,
b) analysing the level of expression or the activity or the sequence of AP5B1 including exons, introns, upstream and downstream non-coding regions involved in the regulation of AP5B1 expression in a biological sample from a healthy individual,
c) comparing the difference in level of expression or activity or in sequence of AP5B1 including exons, introns, upstream and downstream non-coding regions involved in the regulation of AP5B1 expression to healthy individual
d) the difference of step c) is indicative of acne disease or a predisposition to acne, thus diagnosing acne vulgaris.

3. Method for monitoring the progression of acne, comprising the following steps:
a) taking a biological sample from the individual,
b) analysing level of expression or the activity or the sequence of AP5B1 including exons, introns, upstream and downstream non-coding regions involved in the regulation of AP5B1 expression in a sample taken and in which a variation in the expression or activity or in sequence of AP5B1 is an indicator of the progression of acne; progression of acne may be from a predominantly comedonal to a more inflammatory dominated state, it may also mean progression towards specific acne subtypes, like nodulocystic acne or acne conglobata or a progression in the other direction, from a more severe to a less severe form of acne.

4. Method for monitoring the efficacy of a treatment intended for treating acne, comprising the following steps:
a) administering the desired treatment to the individual identified as having one or more of the symptoms of acne,
b) taking a biological sample from the individual,
c) analysing the level of expression or the activity or the sequence of AP5B1 including exons, introns, upstream and downstream non-coding regions involved in the regulation of AP5B1 expression, in which a variation in the expression, activity or sequence of this marker is an indicator in the treatment of acne.

5. In vitro screening method of AP5B1 modulators, comprising determining the capacity of said candidate to restore physiological expression levels and/or biological function, including transactivation activity of AP5B1.

6. In vitro screening method of AP5B1 modulators for the identification of drug candidates, comprising the following steps:
a) Collecting at least one biological sample;
b) Contacting at least one sample or a mixture of samples with one or more drug candidates to be tested;
c) Detecting the expression or biological function of AP5B1 in the biological samples or mixture obtained in b);
d) Selecting drug candidates which are capable of restoring physiological expression levels and/or biological function of AP5B1 measured in said samples or mixtures obtained in b) and comparing the levels with a sample not mixed with the drug candidate.

7. Use of modulators identified by screening methods as defined in claim 6 for the preparation of a composition for treating acne and/or acne associated disorders.
